(19) Europäisches Patentamt
European Patent Office
Office européen des brevets

(11) Publication number: 0 160 136 B1

(12) **EUROPEAN PATENT SPECIFICATION**

(45) Date of publication of patent specification: **21.12.88**

(51) Int. Cl.$^4$: **B 01 J 29/04**, B 01 J 23/74, C 01 B 33/28, C 07 C 1/20

(21) Application number: **84302893.7**

(22) Date of filing: **30.04.84**

# (54) Crystalline encilite composite, its preparation and use.

(43) Date of publication of application: **06.11.85 Bulletin 85/45**

(45) Publication of the grant of the patent: **21.12.88 Bulletin 88/51**

(84) Designated Contracting States: **BE CH DE FR GB IT LI**

(56) References cited:
**EP-A-0 010 572**
**EP-A-0 030 751**
**GB-A-1 555 928**

(73) Proprietor: **Council of Scientific and Industrial Research
Rafi Marg
New Delhi 110 001 (IN)**

(72) Inventor: **Ratnasamy, Paul
National Chemical Laboratory
Poona Maharashtra (IN)**
Inventor: **Borade, Ramesh Babasaheb
National Chemical Laboratory
Poona Maharashtra (IN)**
Inventor: **Kulkarni, Suneeta Balvant
National Chemical Laboratory
Poona Maharashtra (IN)**
Inventor: **Hedge, Suryakant Ganesh
National Chemical Laboratory
Poona Maharashtra (IN)**
Inventor: **Balakrishnan, Ikkandath
National Chemical Laboratory
Poona Maharashtra (IN)**
Inventor: **Rao, Bollapragada Seshagiri
National Chemical Laboratory
Poona Maharashtra (IN)**

(74) Representative: **Spencer, Graham Easdale et al
A.A. Thornton & CO Northumberland House
303-306, High Holborn
London WC1V 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 160 136 B1

**Description**

The present invention relates to a novel crystalline ferrosilicate material, its production and use. More particularly, the invention relates to the novel crystalline ferrosilicate referred to alternatively hereafter as "encilite" or "ferrosilicate encilite" which is active as a catalyst composite. The invention encompasses a process for the production of the novel ferrosilicate encilite catalyst composite and a method for the conversion of alkanols to olefins employing said encilite as catalyst.

Prior art zeolites are exemplified best as crystalline aluminosilicates. These aluminosilicates possess a well ordered porous structure consisting of cavities which are interconnected by a number of channels. The dimensions of the channels and cavities are precisely defined and uniform. As a result, they accept for adsorption molecules of certain dimensions and reject molecules of larger dimension. Due to the aforementioned ability of these materials to discriminate or 'sieve' various molecules depending on their dimensions, these material have also been known as 'molecular sieves'.

A significant feature of the aluminosilicate zeolites is the catalytic activity exhibited by them in those reactions which are catalysed by the presence of Bronsted acid sites. These zeolites possess a rigid framework of cross-linked $SiO_4$ and $AlO_4$ tetrahedra. The negative charge of the tetrahedra containing the trivalent aluminium ion is balanced by the inclusion of a cation, usually an alkali metal ion, as a non-framework charge balancing ion. This non-framework alkali metal cation can be replaced by conventional techniques of ion-exchange by other cations like $NH_4^+$, $Ca^{2+}$, etc. The deammonisation of the zeolites containing the $NH_4^+$ cation yields the acid-form of the zeolite which is active in catalysing reactions like cracking, alkylation or isomerisation.

One limitation of the zeolites of the prior art containing aluminium ions in lattice positions is that due to the high intrinsic acidic strength of the protons associated with the aluminium, the catalytic selectivity of the zeolites in reactions which do not require such very strong acid sites, such as the conversion of methanol to a mixture of ethylene, propylene and butenes is low and decreases with time-on-stream.

In view of this drawback evinced by prior art zeolites when employed in catalytic reactions, efforts have been made to develop a solid zeolite-like material which, instead of aluminium ions, contains other cations in lattice position capable of imparting lower intrinsic acid strength to the associated protonic acid site and thereby increasing the selectivity and service life of the zeolite as a catalyst for the conversion of alkanols, such as methanol, to light olefins.

Crystalline ferrosilicates and methods for their production have been described in GB—A—1555928 and in EP—A—0030751.

It is an object of the present invention to provide an improved crystalline ferrosilicate material having increased selectivity and enhanced stability as a catalyst in the conversion of alkanols to olefins.

A further object of the invention resides in the synthesis of a novel encilite which when employed as catalyst in a catalytic reaction imparts a lower intrinsic acid strength to the associated protonic acid site.

The invention also aims at a process for the preparation of such novel encilite which is active as a catalyst composition material.

A still further object of the invention is a method employing such novel crystalline ferrosilicate encilite as a catalyst for the conversion of alkanols to light olefins.

According to one aspect of the present invention, there is provided a crystalline encilite characterised in that it shows an X-ray powder diffraction pattern having reflection lines as shown below:

| d | I/Ig |
|---|---|
| 11.0420 | 83.30 |
| 9.9945 | 62.57 |
| 9.7096 | 15.90 |
| 7.4057 | 10.22 |
| 7.0752 | 6.93 |
| 6.6713 | 7.95 |
| 6.3203 | 11.36 |
| 5.9804 | 10.22 |
| 5.6755 | 9.19 |
| 5.5345 | 9.09 |
| 5.3679 | 3.40 |
| 5.1038 | 3.40 |
| 4.9087 | 5.68 |
| 4.5950 | 6.25 |
| 4.4357 | 3.40 |
| 4.3496 | 9.09 |
| 4.2467 | 15.99 |
| 4.0733 | 5.68 |
| 4.0080 | 9.09 |
| 3.8371 | 100.00 |
| 3.7416 | 34.09 |
| 3.7047 | 46.59 |
| 3.6449 | 31.81 |
| 3.4293 | 11.36 |
| 3.3285 | 5.50 |
| 3.2406 | 4.54 |
| 3.1291 | 2.84 |
| 3.0394 | 10.79 |
| 2.9378 | 5.60 |

where d is the interplanar spacing (Angstroms) and I/Ig are the relative intensity values; in that it gives infrared framework vibration frequencies as shown below:

| Frequency ($cm^{-1}$) | Intensity |
|---|---|
| 455 | MS |
| 550 | MS |
| 590 | M |
| 620 | M |
| 678 | M |
| 730 | M |
| 800 | MS |
| 870 | MS |
| 888 | MS |
| 1040 | S |
| 1100 | S |
| 1230 | MS |

where

| | |
|---|---|
| VW (very weak) | Less than 10% absorption |
| W (weak) | 10—19% absorption |
| M (medium) | 20—39% absorption |
| MS (medium strong) | 40—69% absorption |
| S (strong) | 70—89% absorption; |

and in that the formula representing the composition of the encilite, expressed in moles of the oxides, is

$$1.0\pm0.2\ M_2O:Fe_2O_3:30\text{—}300\ SiO_2:ZH_2O,$$

where M is a monovalent cation and Z is a value from 0 to 20.

The present invention also provides in a second aspect a process for the preparation of a crystalline encilite as described in the first aspect, comprising reacting in aqueous medium an iron compound, a silicon compound, and a tetraalkyl ammonium salt, characterised in that the aqueous medium also

contains sulphuric acid, and in that the resulting gel is maintained at a temperature between 100—200°C for a period of 1 to 15 days.

Preferably, the monovalent cation M in the composition of the ferrosilicate may be sodium, ammonium, hydrogen or a mixture of any of these.

The ferrosilicate encilite resulting from the process may, according to a preferred feature of the invention, be treated further in order to improve its selectivity and stability as a catalyst composite by subjecting the calcined material to ion exchange with an ammonium salt to obtain encilite having a molar ratio of sodium oxide to iron oxide in the range of from 0.05 to 0.3.

In the specific embodiment when M is sodium, the ion-exchange to which the ferrosilicate encilite is subjected is effective in replacing substantially the sodium ions in the encilite by $NH_4$ ions. The product thus obtained is then further calcined in air, preferably at a temperature of about 400°C, to form a hydrogen ion solid composite.

Preferably, the reaction to form the gel and provide the requisite cations for the synthesis of the encilite is effected by employing a tetraalkyl ammonium salt of the formula:

$$R^1{}_xR^2{}_yN^+A$$

wherein A is a bromide ion, $R^1$ and $R^2$, which may be the same or different, are ethyl, propyl or butyl and x and y, which may be the same or different, are values between 1 and 3, the sum of the values of x and y being equal to 4.

In accordance with a further feature of the invention, the aqueous reaction medium for formation of the gel has the following composition in terms of mole ratios:

| | |
|---|---|
| Silicon (as silica) to iron oxide | 30—200 |
| Sodium oxide to iron oxide | 2—10 |
| Tetraalkyl ammonium salt to iron oxide | 1—20 |
| Sulphuric acid to iron oxide | 1—5 |
| Water to iron oxide | 500—5000 |

By regulation of the quantity of iron in the synthesis medium, it is possible to vary the silicon to iron molar ratio in the final product in a range of from about 30 to about 300.

Typical sources of iron which may be employed in the process of the present invention include the sulfates, nitrates, oxides and hydroxides of iron in various structural and textural forms.

Examples of the sources of silicon employable in the inventive process include silicic acid and silicon oxides in various physical modifications such as colloidal silicon, fumed silica, chromatographic silica, silica gel and sodium silicate.

Examples of the alkali metal cations which may be employed in the process of the invention include the hydroxides, nitrates and sulphates of alkali metals.

Organic templates useful in process of this invention are tetraalkyl ammonium cations of the formulae

$$R^1{}_xR^2{}_yN\ Br \qquad \text{or} \qquad R^1{}_xR^2{}_yNOH$$

wherein $R^1$, $R^2$, x and y have the meanings stated above. Preferred examples of such alkyl ammonium compounds include triethyl-n-propyl ammonium bromide, tetrapropyl ammonium bromide, and triethyl n-butyl ammonium bromide.

It has been found that the presence in the reaction medium of alkyl ammonium ions of the type described along with alkali cations like Na or K has a beneficial effect on the course of the nucleation and crystallisation of the desired encilite material. Moreover, it has been found that the absence of such ammonium ions in the reaction medium during crystallisation gave rise to a solid product which did not have a stable prolonged activity for the conversion of alkanols to hydrocarbons like light olefins.

Preferably, the reaction to produce the gel is effected at a temperature in the range of from 100°C to 200°C over a period of from 1 to 15 days. Conveniently, the reaction is carried out in an autoclave reactor under autogeneous pressure of steam in the above-mentioned temperature range.

From the point of view of its physical capability to act as a molecular sieve, any zeolite is ascertained and evaluated in terms of its pore structure, i.e. the amount of water, n-hexane, cyclohexane or 2-methylpentane that can be absorbed in the porous cavities of the material at 24°C. While the extent of hydrophilicity of the porous cavities is indicated by the amount of water adsorbed, the adsorption of n-hexane may be used to evaluate the hydrophobic nature of the internal source of the zeolite material as well as the total internal void volume of the said material. A comparison of the respective amounts of adsorption of n-hexane, neopentane, benzene and cyclohexane gives an indication of the diameter of the

pores in the crystalline ferrosilicate since the three molecules mentioned hereinbefore differ mainly in their cross-sectional dimensions and otherwise possess a similar hydrophobic character.

In order to assess the pore structures of the ferrosilicate of the present invention, an encilite sample was degassed at 400°C for 4 hours at a pressure lower than $10^{-8}$ bar. The sample was then cooled to 24°C. The sorbate was then admitted and the amount adsorbed at equilibrium was noted. It was invariably found that for the crystalline ferrosilicate prepared according to the present invention, the amount of n-hexane adsorbed per unit weight of solid was always higher than the amount of water adsorbed indicating thereby a highly hydrophobic nature of the internal surface within the pores of the encilite. The hydrophilicity, as measured by the amount of water adsorption and the surface acidity, as measured by the amount and strength of $NH_3$ adsorption on the encilite, was intermediate between the high values observed in the case of aluminosilicate zeolites and the very low values observed in the case of polymorphs of $SiO_2$. The ferrosilicate encilite of this invention thus comprises a molecular sieve with an adsorption pore size near $6\times10^{-10}$ m (6Å). At 24°C, it adsorbs molecules as large as benzene (kinetic diameter=$5.9\times10^{-10}$ m (5.9 Å)) but rejects molecules larger than $6\times10^{-10}$m (6Å) such as neopenzane (kinetic diameter=$6.2\times10^{-10}$m (6.2Å)).

In terms of its chemical structure, the encilite produced by the process of the present invention has a silica to iron oxide molar ratio of at least 30 but not more than 300, and a sodium oxide to iron oxide molar ratio of at least 0.7 but not more than 1.3. The iron ions in the encilite are in the trivalent positive oxidation state. This structure enables the encilite produced by the present invention to evince a number of advantageous characteristics which are discussed hereafter.

First of all, there is the unique stability of its trivalent iron ions under conditions of ion-exchange. The reason for this lies in the fact that such trivalent iron ions $Fe^{3+}$, are incorporated within the crystalline framework lattice of the encilite itself and are not simply present as non-framework cations in the intra-crystalline void volume where they could be easily replaced by other cations through conventional techniques of ion-exchange.

Secondly, the trivalent iron ions present in the encilite of the present invention are uniquely stable against reduction. Once again, this is due to their incorporation within the crystalline framework lattice and their absence as non-framework cations in the intra-crystalline void volume where they could easily have been reduced to divalent or lower oxidation states (FeO or $Fe_3O_4$) and thence to metallic iron by conventional hydrogen reduction.

The trivalent iron ions incorporated in the tetrahedral lattice framework of the encilite have been subjected to electron spin resonance spectroscopy. This shows them to evince a strong absorption band at g=4.3 characteristic of $Fe^{3+}$ surrounded by four oxygen anions at the corners of a tetrahedron. This observation confirms that the trivalent iron ions in encilite are indeed present within the tetrahedral zeolitic lattice and not in non-framework positions, where they would have been surrounded by six oxygen anions at the corners of an octahedron and hence would not exhibit the absorption band at g=4.3 in the electron spin resonance spectrum.

The encilite of this invention has an exceptionally high degree of thermal stability up to 1000°C, thereby rendering it especially suitable for use as a catalyst in processes involving elevated temperatures. It would appear that the presence in the reaction mixture of both sodium and mixed alkyl ammonium ions in addition to the oxides of silicon and iron is necessary to obtain solid encilite possessing the desired catalytic activity. Even though the precise mechanism by which alkyl ammonium ions, when present in the reaction medium, influence the rate and course of the crystallisation of the desired catalytic mass is not known in detail, in may be possible to speculate that they influence the structure of water in the vicinity of the reacting $SiO_4$ and Fe tetrahedral species in such a way that nucleation and crystallisation occur leading to a product, i.e. encilite, which possesses both a desirable porous structure and which is active, selective and stable in the catalytic conversation of alkanols to olefins.

Accordingly, the present invention further provides in a third aspect a method for converting an alkanol or an aqueous alkanol mixture into hydrocarbons consisting primarily of light olefins, characterised in that it comprises contacting in the vapour phase under a conversion reaction temperature from 300°C to 500°C, a feed comprising one or more monohydric alcohols having 1 to 4 carbon atoms, or a mixture thereof with water over a crystalline encilite produced by a process as described in the second aspect where the ferrosilicate encilite is in the form of a hydrogen ion solid composite.

According to a specific embodiment of the method in question, the encilite used is prepared by reaction in aqueous medium of iron, silicon, an alkali metal and sulphuric acid with a tetraalkyl ammonium salt of the formula:

$$R^1_xR^2_yN^+A$$

wherein A is a bromide or hydroxide ion, $R^1$ and $R^2$, which may be the same or different, are alkyl groups containing from 2 to 4 carbon atoms and x and y, which may be the same or different, are values between 1 and 3, the sum of the values of x and y being equal to 4, followed by heating the gel resulting from said reaction, recovery of the solid residue and calcination of the recovered solid.

Conveniently, the ferrosilicate encilite catalyst is combined with a binder in order to impart greater mechanical strength thereto during its employment in the alkanol conversion. Examples of typical binders

include silicon, alumina, bentonite, kaolinite of admixtures thereof. The ferrosilicate encilite is combined with such binder in an amount of from 50% to 95% by weight of catalyst based on the combined weight of catalyst and binder. When the binder is a clay-like material such as alumina, bentonite or kaolinite, a blend of catalyst and binder with water can be prepared to the desired consistency and the blend formed into a convenient shape, for instance by extrusion. The shape or extrudate is then dried before being employed in the method of conversion.

The alkanol most suited for conversion by the method of the present invention is methanol but other alkanols having up to 4 carbon atoms can also be employed. In a specific embodiment of the method, the catalytic encilite is located within a downflow integral fixed bed reactor and vapours of methanol or other alkanol are passed over it at a weight hourly space velocity (WHSV) of 2 to 20.

The structural features of the ferrosilicate encilite produced by the process of the present invention and employed in the alkanol conversion method described above can be ascertained by a variety of techniques. The findings listed in Table 1 hereafter were obtained by x-ray powder diffraction measurements on a Phillips PW 1730 diffractometer using copper K α-radiation in conjunction with a nickel filter. In such Table, the interplanar spacings are represented by 'd' and are expressed in terms of Angstrom units (A) and the relative intensity values are represented by $I/I_0$ and are expressed in terms of the relative peak heights. The framework vibration frequencies (in $cm^{-1}$) of encilite containing iron cations in lattice positions are shown in Table 2. The infrared spectra of encilite were recorded with a Perkin-Elmer infrared spectrometer.

TABLE 1
X-ray diffraction pattern of encilite-sodium form

| d | I/Ig |
|---|---|
| 11.0420 | 83.30 |
| 9.9945 | 62.57 |
| 9.7096 | 15.90 |
| 7.4057 | 10.22 |
| 7.0752 | 6.93 |
| 6.6713 | 7.95 |
| 6.3203 | 11.36 |
| 5.9804 | 10.22 |
| 5.6755 | 9.19 |
| 5.5345 | 9.09 |
| 5.3679 | 3.40 |
| 5.1038 | 3.40 |
| 4.9087 | 5.68 |
| 4.5950 | 6.25 |
| 4.4357 | 3.40 |
| 4.3496 | 9.09 |
| 4.2467 | 15.99 |
| 4.0733 | 5.68 |
| 4.0080 | 9.09 |
| 3.8371 | 100.00 |
| 3.7416 | 34.09 |
| 3.7047 | 46.59 |
| 3.6449 | 31.81 |
| 3.4293 | 11.36 |
| 3.3285 | 5.50 |
| 3.2406 | 4.54 |
| 3.1291 | 2.84 |
| 3.0394 | 10.79 |
| 2.9378 | 5.60 |

TABLE 2
Infrared framework vibration frequencies

| Frequency ($cm^{-1}$) | Intensity |
|---|---|
| 455 | MS |
| 550 | MS |
| 590 | M |
| 620 | M |
| 678 | M |
| 730 | M |
| 800 | MS |
| 870 | MS |
| 888 | MS |
| 1040 | S |
| 1100 | S |
| 1230 | MS |

For appreciating the absorption at the intensity set out in Table 2, the following explanation is given:

| Intensity | Absorption (%) |
|---|---|
| VW (very weak) | Less than 10 |
| W (weak) | 10—19 |
| M (medium) | 20—39 |
| MS (medium strong) | 40—69 |
| S (strong) | 70—89 |

It should be understood that the x-ray diffraction and infrared framework vibration frequencies are characteristics of all species of encilite. Ion exchange of the sodium ions with other cations like $H^+$, $NH_4^+$, $K^+$, $Ca^{2+}$ reveals substantially the same pattern with some minor shifts in interplanar spacings or vibration frequencies and variation in the relative intensity of the various peaks. Other minor variations can occur depending on the silicon to iron ratio of the particular sample as well as on the prolonged duration of its subjection to steam treatment at elevated temperatures.

The invention in both its aspects will now be described in greater detail in the following Examples which describe non-limitatively the preparation of the novel encilite and its use in the conversion of alkanols to light hydro carbons. Where adsorption data are given, such data have been measured gravimetrically in a high vacuum adsorption system using a quartz spring balance.

Example 1

This example illustrates the preparation of the ferrosilicate encilite. To 20 gms. of sodium silicate ($8.2\times Na_2O$, $27.2\times SiO_2$, $26.9\times H_2O$) 10 ml. of water were added to constitute solution A. 2.5 gms. of tetrapropyl ammonium bromide were dissolved in 10 ml. of water to yield solution B. 0.54 gms. of ferric sulphate (31.2% $Fe_2O_3$) was added to water and heated to 50°C to yield a clear solution C. 1.76 gms. of $H_2SO_4(98\times)$ were diluted in 15 m. of water to produce solution D. Solutions B was added to solution A with stirring. Solution C was then added to the mixture with stirring. Finally, solution D was added to the resulting mixture dropwise with constant vigorous stirring. A solid gel was formed whose pH was about 10.2. The gel was heated at 180°C in a closed autoclave under autogeneous pressure of steam for 1 day. After this process, the autoclave was quenched in cold water. The autoclave was opened and the supernatant liquid separated from the solid product by filtration. The pH of the supernatant liquid was 11.8. The solid product was washed with hot water till the washings were free from sulphate ions. The solid was then dried in air in an oven at 120°C for 12 hrs. and calcined in air at 520°C for 8 hours to yield a solid crystalline material.

The x-ray diffraction pattern of the material is set forth in Table 1. The infrared framework vibration frequencies of the material are given in Table 2. The chemical composition of the solid material was found to be 0.8 $Na_2O$: $Fe_2O_3$: 71$SiO_2$. The electron spin resonance spectrum of this material was found to contain a sharp absorption band at g=4.3.

Example 2

The procedure of Example 1 was repeated using triethyl-n-propyl ammonium bromide instead of tetrapropyl ammonium bromide. The solid material that was obtained exhibited an x-ray diffraction pattern

that was substantially the same as that set forth in Table 1. The framework vibration frequencies in the infrared region were also identical to that given in Table 2. The chemical composition of the solid material was found to be 0.92 $Na_2O$:$Fe_2O_3$:76$SiO_2$.

Example 3

This example illustrates the process for replacing the sodium ions in the crystalline ferrolisicate by ammonium ions. Samples of the product of Example 1 were subjected to ion-exchange. The solid was treated twice with an aqueous solution of 5N ammonium chloride at 90°C for 15 hours each time. After each exchange, the product was washed with hot distilled water till the filtrate was free from chloride ions. The product thus obtained was dried at 120°C in air in an oven to yield the ammonium form of encilite. The molar ratio of sodium oxide to iron oxide in the material was 0.04. By varying the concentration of $NH_4Cl$ and the number of exchanges, the sodium oxide ratios could be varied from 0.3 to 0.05.

Example 4

This Example illustrates the process for replacing the ammonium ions in the crystalline ferrosilicate by hydrogen ions. Samples of the product of Example 3 were calcined at 520°C for 10 hours in a stream of dry air to yield the hydrogen form of encilite.

Example 5

A silicate solution was made by mixing 500 g sodium silicate (27.2% $SiO_2$, 8.4% $Na_2O$ and 63.9% $H_2O$), 87.2 g. dipropyl-diethyl bromide and 400 g of water. An acid solution was made by mixing 21.8 g. of ferric sulphate hexahydrate, 70.4 g $H_2SO_4$ and 1600 g. of water. This acid solution was then added to the silicate solution slowly under vigorous stirring to form a firm gel. The pH of the gel was measured to be 10.2. The gel had the following composition in terms of mole ratios of oxides:

$$4.38\ H_2O:\ 25.5\ Na_2O:\ Fe_2O_3:\ 83\ SiO_2:\ 3365\ H_2O.$$

where R is the dispropyl-diethyl cation.

The resulting gel was charged into a one gallon autoclave. The crystallization was carried out at 150°C without agitation for 130 hours. The autoclave was quenched in water to cool it at room temperature. The water-washed and dried 200 g. product was found to be a crystalline ferrosilicate characterized by x-ray diffraction (XRD) data as given in Table 1.

Example 6

The identical mixing sequence of Example 1 was followed and apart from a change in the cation of quarternary ammonium compound, the other reaction components were the same as Example 5. A gel was formed by mixing the following reaction components:

i) 800 g. sodium silicate
ii) 94.7 g. tripropyl-ethyl bromide+400 g. $H_2O$
iii) 70.4 g $H_2SO_4$+1200 g $H_2O$.

This gel had the following composition in terms of mole ratios of oxides:

$$4.38\ R_2O:\ 25.5\ Na_2O:\ Fe_2O_3\ 83\ SiO_2:\ 3365\ H_2O$$

where R is the tripropylethyl cation.

The thick homogeneous gel mix was transferred to a one gallon autoclave and crystallized at 180°C without agitation for 48 hours. After quenching the autoclave to room temperature, the product was filtered, washed and dried. The product (200 g) exhibited a similar XRD pattern to that given in Table 1, confirming it to be a crystalline ferrosilicate.

Example 7

An encilite catalyst extrudate with increased mechanical strength was prepared by treating and mixing hydrogen forms of encilite ferrosilicate zeolite powder saturated with $H_2O$ vapour with bentonite clay at room temperature and shaping the mass by passing it through a screw driven steel-die with a 1/8 inch opening. The optimum content of bentonite was 15 percent on dry basis. The extrudates were kept open to the atmosphere for 2 to 4 hours, dried in air in an oven at 100°C—100°C for about 4 hours and finally calcined in a muffle furnace at 550°C for 10 hours.

Example 8

In this example 1/8 inch extrudates of encilite catalyst were prepared by mixing hydrogen forms of encilite ferrosilicate zeolite powder with alumina binder at room temperature. The optimum content of alumina binder used was 50 percent on dry basis. The encilite extrudates were dried in air in an oven at 100°C—110°C and then calcined in a muffle furnace at 550°C for 10 hours before putting them to use.

Example 9

A solution was prepared by mixing 348.4 g of colloidal silicon dioxide (96.46% $SiO_2O$. 3.77% $H_2O$), and 609 ml tetrapropyl ammonium hydroxide (40% aqueous solution) in 200 g of water. The resulting mixture was stirred for 15 minutes. To this, a solution of 81.28 g of ferric sulphate hexahydrate in 350 g. of water was added slowly under stirring. An alkali solution of 20.48 g. of sodium hydroxide in 675 g. of water was added to the prepared mixture slowly under stirring to form a homogeneous gel. The pH of the g measured to be 12.5. The gel had the following composition in terms of mole ratios of oxides:

$$3.75\ R_2O:\ 1.6\ Na_2O:\ Fe_2O_3:\ 35\ SiO_2:\ 425\ H_2O$$

where R is the tetraalkyl ammonium ion.

This gel was charged into a one gallon capacity stainless steel autoclave. The crystallization was carried out at 180°C without agitation for 140 hours. The autoclave was quenched in water and cooled to room temperature. The water-washed and dried product (340 g) was found to be a crystalline ferrosilicate characterized by an x-ray diffraction pattern as shown in Table 1.

Example 10

In this example keeping the identical mixing sequence and procedure of Example 1, a gel was formed by mixing the following components:

i) 560 g colloidal silicon dioxide+55 g $H_2O$
ii) 153 ml. tetrapropylammonium (TPA) hydroxide (40% aq).
iii) 20.32 g $Fe_2(SO_4)_3 \cdot 6H_2O$+75 g $H_2O$
iv) 5.12 g NaOH+175 g $H_2O$.

The gel so formed had the following composition in terms of mole ratios of oxides:

$$3.75\ R_2O:\ 1.6\ Na_2O:\ Fe_2O_3:\ 225\ SiO_2:\ 425\ H_2O$$

where R is a tetrapropyl ammonium ion.

The resulting homogeneous gel was charged into a one gallon capacity stainless steel autoclave. The crystallization was carried out at 180°C without agitation for 140 hours. The autoclave was quenched in water and cooled to room temperatures. The water-washed and dried product (550 g) was found to be crystalline ferrosilicate characterized in by XRD data as given in Table 1.

Example 11

In this example keeping the identical mixing sequence and procedure of Examples 1, and 2, a gel was formed by mixing the following components:

i) 373 g. colloidal silicon dioxide+160 g. $H_2O$
ii) 305 ml TPA-OH (40% aq. solution)
iii) 40.64 g $Fe_2(SO_4)_3$ $6H_2O$+150 g. $H_2O$
iv) 10.24 g NaOH+300 g $H_2O$

The gel formed had following composition in terms of mole ratios of oxides:

$$3.75\ R_2O:\ 1.6\ Na_2O:\ Fe_2O_3:\ 75\ SiO_2:\ 425\ H_2O$$

where R is a tetrapropyl ammonium ion.

The resulting homogeneous gel mix was charged into a one gallon capacity stainless steel autoclave. The crystallization was carried out at 180°C without agitation for 140 hours. The washed and dried product (370 g.) was found to be a crystalline ferrosilicate characterized by XRD data given in Table 1.

Example 12

402 g chromatographic silica gel (90.2%) $SiO_2$, 9.8% ($H_2O$) were transferred to a stainless steel autoclave of one gallon capacity. A solution containing 169.3 g of tetra propyl ammonium bromide in 1000 g $H_2O$ was added to chromatographic silica gel in the autoclave. To this slurry, 45.4 g sodium hydroxide in 1000 g water was then added with stirring to yield a homogeneous gel. An acid solution of 42.3 g ferric sulphate hexahydrate in 1387 g water was finally added slowly with vigorous stirring to form a gel mix. The stirring was continued for another 30 minutes to yield a homogeneous mix, the pH of which was recorded to be 10.6. The gel has the following composition in terms of mole ratios of oxides.

$$3.82\ R_2O:\ 6.83\ Na_2O:\ Fe_2O_3:\ 72.5\ SiO_2:\ 2270\ H_2O:$$

The autoclave was then closed and the crystallization was carried out at 180°C without agitation for 70 hours. The autoclave was quenched in water and cooled down to room temperature. The water-washed and dried product (400 g) was found to be a crystalline ferrosilicate characterized by XRD data as given in Table 1.

Example 13

In this example keeping the identical mixing sequence and procedure of Example 4, a gel mix was formed by mixing together the following components:

i) 201 g chromatographic silica-gel
ii) 169.3 g TPA-Br+1000 g $H_2O$
iii) 45.3 g NaOH+1000 g $H_2O$
iv) 42.3 g $Fe_2(SO_4)_36H_2O$+1387 g. $H_2O$

The gel so formed had the following composition in terms of mole ratios of oxides:

$$3.82\ R_2O: 4.82\ Na_2O_3: Fe_2O_3: 36\ SiO_2: 1136\ H_2O$$

where R is a tetrapropyl cation.

The gel mix showed a pH of 10.85. The gel was crytallized in an S.S. autoclave (1 gallon capacity) at 180°C without agitation for 90 hours. The autoclave was quenched as usual to cool it to room temperature. The water-washed and dried product (195 g) was found to be a crystalline ferrosilicate characterized by XRD data as given in Table 1.

Example 14

A silicate solution was made by mixing 800 g sodium silicate (27.2% $SiO_2$, 8.4% $Na_2O$, and 63.9% $H_2O$), 84.2 g triethyl-n-propyl ammonium bromide and 400 g water. An acid solution was made by mixing 21.8 g $Fe_2(SO_4)_3$ $6H_2O$, 70.4 g $H_2SO_4$ and 1600 g water. This acid solution was then added to the silicate solution under vigorous stirring to form a thick gel. The pH of the gel measured 10.5. The gel had the following composition in terms of mole ratios of oxides:

$$4.38\ R_2O: 25.35\ Na_2O: Fe_2O_3: 83\ SiO_2: 3365\ H_2O$$

where R is the triethyl-n-propyl cation.

The resulting gel was charged into a one gallon capacity S.S. autoclave. The crystallization was carried out at 180°C without agitation for 49 hours. The autoclave was quenched as usual to cool it to room temperature. The water-washed and dried product (200 g.) was found to be a crystalline ferrosilicate characterised by XRD data as given in Table 1. The chemical composition of the hydrated product was analysed as $Fe_2$: 72 $SiO_2$: 0.8 $Na_2O$: $10H_2O$.

Example 15

In this example, keeping the identical mixing sequence and procedure of Example 6, a gel was formed by mixing the following reaction components:

i) 800 g sodium silicate
ii) 84.2 g of triethyl-n-propyl ammonium bromide
iii) 50.0 g $Fe_2(SO_4)_3$ $6H_2O$+500 g $H_2O$
iv) 56.08 g $H_2SO_4$+1000 g $H_2O$

The gel formed had the following composition in terms of mole ratios of oxides:

$$4.38\ R_2O: 25.35\ Na_2O: Fe_2O_3: 36\ SiO_2: 3365\ H_2O$$

The resulting gel was charged into a one gallon S.S. autoclave and crystallized at 180°C without agitation for 96 hours. The product (200 g) was found to crystalline ferrosilicate.

Example 16

In another example, keeping the identical mixing sequence and procedure of Examples 6 and 7, a gel was formed by mixing the following reaction components:

i) 800 g sodium silicate (27.2% $SiO_2$, 8.4% $Na_2C$)
ii) 84.2 g of trientyl-propyl ammonium bromide+500 g $H_2O$
iii) 8.0 g $Fe_2(SO_4)_3$ $6H_2O$+500 $H_2O$
iv) 78.4 g $H_2SO_4$+1000 g $H_2O$

The gel formed had the following composition in terms of mole ratios of oxides:

$$4.38\ R_2O: 25.35\ Na_2O: Fe_2O_3: 226\ SiO_2: 3365\ H_2O$$

The gel mix was crystallized at 180°C without agitation in a one gallon S.S. autoclave for 30 hours. The autoclave was quenched and cooled down to room temperature as usual. The water-washed and dried product (200 g.) was found to be a crystalline ferrosilicate characterized by XRD data as given in Table 1.

Example 17

A silicate solution was made by mixing 800 g. sodium silicate (27.2% $SiO_2$, 8.4% $Na_2O$, and 63.9% $H_2O$), 100 g tetrapropyl ammonium bromide and 400 g of water. An acid solution was made by mixing 9.28

g of $Fe_2(SO_4)_3$ $6H_2O$, 84 g $H_2SO_4$ and 1600 g of water. This acid solution was then added to the silicate solution slowly under vigorous stirring to form a thick gel. The pH of the gel was measured to be 10.5. The gel had the following composition in terms of mole ratios of oxides:

$$10\ R_2O:\ 58\ Na_2O:\ Fe_2O_3:\ 194\ SiO_2:\ 7492\ H_2.$$

The resulting gel was charged into a one gallon capacity S.S. autoclave. The crystallization was carried out at 180°C, without agitation for 29 hours. The autoclave was quenched in water and cooled to room temperature. The water-washed and dried product (200 g) was found to be a crystalline ferrosilicate characterized by an X-ray diffraction pattern as given in Table 1.

Example 18

In this Example keeping the identical mixing sequence and procedure of Example 9, a gel was formed by mixing together the following reaction compounds:

i) 800 g sodium silicate
ii) 100 g of tripropylammonium bromide+400 g $H_2O$
iii) 12.4 g $Fe_2(SO_4)_3$ $6H_2O$+800 g $H_2O$
iv) 73.6 g $H_2SO_4$+800 g $H_2O$

The gel so formed had the following composition in terms of mole ratios of oxides:

$$7.75\ R_2O:\ 44.7\ Na_2O:\ Fe_2O_3:\ 150\ SiO_2:\ 5576\ H_2O$$

The resulting homogeneous gel was charged into a one gallon capacity S.S. autoclave. The crystallization was carried out at 180°C without agitation for 20 hours. The autoclave was quenched in water as usual. The water-washed and dried product (200 g) was found to be a crystalline ferrosilicate characterized by XRD data as given in Table 1.

Example 19

In this example keeping the identical mixing sequence and procedure of Examples 9 and 10, a gel was formed by mixing the following reaction components:

i) 800 g sodium silicate+400 g $H_2O$
ii) 100 g TPA-Br+400 g $H_2O$
iii) 06.1-g $Fe_2(SiO_4)_3$ $6H_2O$+400 g $H_2O$

The gel so formed had the following composition in terms of mole ratios of oxides:

$$15.61\ R_2O:\ 90\ Na_2O:\ Fe_2O_3:\ 300\ SiO_2:\ 11630\ H_2O$$

The thick homogeneous gel was transferred to a one gallon S.S. autoclave and crystallized at 180°C for 15 hours without agitation. After quenching the autoclave to room temperature as usual, the product was water-washed and dried. The product (200 g) exhibited an XRD pattern similar to that given in Table 1, confirming it to be a crystalline ferrosilicate.

Example 20

In this example, keeping the same sequence of mixing as the previous Examples, a gel was formed with the following reaction components:

i) 800 g sodium silicate+300 g $H_2O$
ii) 100 g TPA-Br+500 g $H_2O$
iii) 61.6 g $Fe_2(SO_4)_3$ $6H_2O$+400 g $H_2O$
iv) 64 g $H_2SO_4$+800 g $H_2O$

The gel so formed had the following composition in terms of mole ratios of oxides:

$$1.56\ R_2O:\ 9\ Na_2O:\ Fe_2O_3:\ 30\ SiO_2:\ 1163\ H_2O$$

The gel was charged into a one gallon S.S autoclave and crystallized at 180°C without agitation for 130 hours. The water-washed product after quenching the autoclave to room temperature was dried in air in an oven at 110°C for 4 hours. The product (200 g) was found to be a crystalline ferrosilicate characterized by XRD data as given in Table 1.

Example 21

In this example keeping the same $SiO_2/Fe_2O_3$ ratio, i.e. 30, as in Example 20 with the same quantity of reactants except that the quantity of $H_2SO_4$ was reduced to half, a gel mix was formed. The pH of the gel was measured to be 11.0. This gel was then crystallized as usual in a one gallon S.S autoclave at 180°C without agitation for 110 hours. The water-washed and dried product (200 g) was found to be a crystalline ferrosilicate.

Example 22

A solution was made by mixing 168 g colloidal silicon dioxide (96.46% $SiO_2$, and 3.72% $H_2O$), 45 g tetra propylammonium bromide and 400 g water. To this solution, 575 ml. liquor ammonia (25% $NH_3$) and 15.48 g potassium hydroxide were added under stirring. An acid solution was made by mixing 7.48 g $Fe_2(SO_4)_3$ $6H_2O$, 52.8 g $H_2SO_4$ and 1600 g of water. This acid solution was then added to the first solution slowly under vigorous stirring to form a thick gel. The gel had the following composition in terms of mole ratios of oxides:

$$9.66\ H_2O: Fe_2O_3: 185\ SiO_2: 287\ (NH_4)_2O: 9.4\ K_2O: 3292\ H_2O$$

The resulting gel was charged into a one gallon S.S. autoclave. The crystallization was carried out at 150°C without agitation for 150 hours. The autoclave was quenched in water and cooled to room temperature. The water-washed and dried product (42 g) was found to be a crystalline ferrosilicate with XRD data as given in Table 1.

Example 23

A silicate solution was made by mixing 600 g sodium silicate (27.2% $SiO_2$, 8.4% $Na_2O$ and 63.9% $H_2O$), 72.8 g tetrapropylammonium bromide and 400 g of water. To this, 575 ml. liquor ammonia (25% $NH_3$) and 400 g of water were added slowly under stirring. An acid solution was made by mixing 7.48 g $Fe_2(SO_4)$ $6H_2O$, 80 g $H_2SO_4$ and 1600 g water. This acid solution was then added slowly under vigorous stirring to first solution to form a homogeneous gel. The gel had the following composition in terms of mole ratios of oxides:

$$9.8\ R_2O: 57\ Na_2O: Fe_2O_3: 190\ SiO_2(NH_4)_2O: 1092\ H_2O$$

The resulting gel was charged into a one gallon S.S. autoclave and crystallized at 150°C without agitation for 115 hours. The water-washed and dried product (150 g) was found to be a crystalline ferrosilicate.

Example 24

In this example, keeping the same $SiO_2/Fe_2O_3$ i.e. 190, as in Example 23 with the same quantity of reactants but without liquor ammonia, a gel was formed. This gel was then crystallized as usual in a one gallon S.S. autoclave at 200°C without agitation for 16 hours. The water-washed and dried product (150 g) exhibited the similar XRD pattern as reported in Table 1.

Example 25

In this example keeping the same mixing sequence and procedure as in Examples 9, 10 and 11, a gel was formed by combining the following reaction components:

i) 800 g sodium silicate
ii) 100 g TPA-Br+400 g $H_2O$
iii) 21-6 g $Fe_2(SO_4)_3$ $6H_2O$+400 g $H_2O$
iv) 70.4 g $H_2SO_4$+1200 g $H_2O$

The gel so formed had the following composition in terms of mole ratios of oxides:

$$4.43\ R_2O: 25.5\ Na_2O: Fe_2O_3: 85\ SiO_2: 3292\ H_2O$$

The gel mix was crystallized as usual in a one gallon S.S. autoclave at 200°C without agitation for 16 hours. The product (200 g) exhibited the similar XRD pattern as given in Table 1, confirming it to be a crystalline ferrosilicate.

Example 26

In this example, the same molar ratio of $SiO_2/Fe_2O_3$ as previous Examples and other conditions were maintained unchanged except that crystallization was carried out at 180°C without agitation for 48 hours. The product (200 g.) exhibited the similar XRD pattern as given in Table 1, confirming it to be a crystalline ferrosilicate.

Example 27

In this example, the same sequence of mixing and the same composition of the gel in terms of mole ratios of oxides as in example 21 was maintained except that the gel was crystallized at 160°C without agitation for 72 hours. The product (200 g) was found to be a crystalline ferrosilicate. The chemical composition of the water-washed, dried and activated sample was analyzed to be:

| | | |
|---|---|---|
| $SiO_2$ | : | 92.1% wt. |
| $Fe_2O_3$ | : | 3.31% wt. |
| $Na_2O$ | : | 1.06% wt. |
| $H_2O$ | : | 3.43% wt. |

Example 28

In this example, the parameters, reactants and molar ratios of Examples 18 and 19 are maintained except that the crystallization was carried out at 140°C without agitation for 96 hours. The product (200 g) was found to be a crystalline ferrosilicate conforming the XRD data as given in Table 1.

Example 29

A silicate solution was made by mixing 800 g sodium silicate (27.2% $SiO_2$, 8.4% $Na_2O$ and 63.9% $H_2O$), 100 g of tetra propylammonium bromide and 400 g water. An acid solution was made by mixing 21.72 g $Fe_2(SO_4)_3$ $6H_2O$, 70.4 g of $H_2SO_4$ and 1600 g of water. This acid solution was then added to the silicate solution slowly under vigorous stirring to yield a thick gel. The pH of the gel was measured to be 10.25. The gel had the following composition in terms of mole ratios of oxides:

$$4.42\ R_2O: 25.5\ Na_2O: Fe_2O_3: 86\ SiO_2: 3292\ H_2O$$

where R is tetrapropylammonium ion.

The resulting gel was charged into a one gallon S.S. autoclave and crystallized at 180°C without agitation for 48 hours. The autoclave was quenched in water and cooled to room temperature. The water-washed, dried and activated (at 550°C for 10 hours) product (200 g) was found to be a crystalline ferrosilicate characterized by an X-ray diffraction pattern as shown in Table 1.

The adsorption properties of the activated form of encilite are as follows:

| Adsorbate | Temperature | P/P | g/100 g. |
|---|---|---|---|
| Cyclohexane | 24°C | 0.84 | 3.84 |
| n-hexane | -do- | 0.83 | 10.94 |
| Water | -do- | 0.78 | 7.97 |

Example 30

A solution was made by mixing 200 g sodium silicate (27.2% $SiO_2$, 8.4% $Na_2O$ and 63.9% $H_2O$), 49.9 g tripropylamine and 42.1 g n-propyl bromide in 200 g of water. The resulting mixture was stirred for 15 minutes. To this, an acid solution of 20.32 g ferric sulphate hexahydrate and 86.8 g $H_2SO_4$ in 1800 g of water, was added slowly under vigorous stirring to form immediately a firm gel. This gel was then stirred for another 30 minutes to yield a homogeneous mixture. The pH of the gel was measured to be 9.4. The gel had the following composition in terms of mole ratios of oxides:

$$4.29\ R_2O: 27.4\ Na_2O: Fe_2O_3: 88\ SiO_2: 3505\ H_2O.$$

The homogeneous gel thus formed was charged into a one gallon S.S. autoclave and crystallized at 180°C without agitation for 8 days. The autoclave was quenched in water and cooled to room temperature. The water-washed and dried product was found to be a crystalline ferrosilicate characterized by XRD data as given in Table 1.

Example 31

To exemplify a preferred ebmodiment of this invention, a silicate solution was prepared in a 600 ml. capacity S.S. autoclave by mixing 120 gms. of sodium silicate, 13.44 gms of triethyl n-butyl ammonium bromide and 150 gms of water. An acid solution prepared by mixing 3.26 gms of ferric sulphate hexahydrate, 10.56 gms of sulfuric acid and 150 gms of water was added slowly to the silicate solution under vigorous stirring to form a homogeneous gel. The p/H of the gel was 10.0±.2. The autoclave was closed and crystallisation was carried out at 180°C while stirring the mixture of 100 RPM for 36 hours. The autoclave was quenched to room temperature. The pH of the supernatant liquid was 11.6±0.2. The solid product was filtered out from the liquid, water washed and dried at 100°—110°C in air in an oven for 10 hours. The dried product was calcined at 550°C for 4 hours.

The product obtained after calcination was encilite with characteristic XRD pattern as given in Table 1. This was converted to the acid form by the procedure given in Examples 3 and 4. The hydrogen form of encilite thus obtained was mixed with 20% bentonite and 15% kaolinite on dry basis and formed into extrudates by the procedure given in Example 7.

Example 32

Methanol was passed in the vapour phase at atmospheric pressure over an encilite catalyst, the catalyst being located in a downflow integral fixed bed reactor. The vapour was passed at a weight hourly space velocity (WHSV) of 5.1, and in this connection, it is clarified that WHSV is the weight in grams of the reactant, in this case methanol, which is passed over 1 gm. of the catalyst per hour at the specified

temperatures and pressures mentioned hereunder. The products of the reaction were analysed quantitatively by gas chromatography. The results are illustrated in Table 3 hereafter.

TABLE 3
Conversion of methanol to hydrocarbons

| | |
|---|---|
| Feed | 100% methanol |
| WHSV ($hr^{-1}$) | 2.2 |
| Pressure | Atmospheric |
| Temp. | 450°C |
| Conv. of methanol | 100% |
| Product distribution (wt.Z) | |
| Dimethyl ether | Nil |
| Paraffins | |
| $C_1$ | 2.7 |
| $C_2$ | Trace |
| $C_3$ | 5.1 |
| $C_4$ | 7.8 |
| Olefins | |
| $C_2$ | 10.3 |
| $C_3$ | 21.6 |
| $C_4$ | 15.5 |
| $C_5$ and other aliphatics | 20.7 |
| Aromatics | |
| Benzene | 0.7 |
| Toluene | 3.2 |
| Xylene | 7.4 |
| $C_9+C_{10}$ | 5.0 |

Example 33

The effect of temperature on the conversion of methanol to hydrocarbons is illustrated in this example. The experiment and the analysis of the products obtained were carried out as in Example 32. The results are tabulated in Table 4.

TABLE 4
Conversion of methanol to hydrocarbons: effect of temperature

| | | | | | |
|---|---|---|---|---|---|
| Feed | 100% methanol | | | | |
| WHSV ($hr^{-1}$) | 2.2 | | | | |
| Pressure | Atmospheric | | | | |
| Temp. °C | 300 | 365 | 405 | 450 | 500 |
| % conv. methanol | 54.4 | 83.6 | 99 | 100 | 100 |
| Product distribution (Wt.%) | 92.5 | 7.4 | 1.1 | 0 | 0 |
| Dimethyl ether paraffins $C_1$ | 0.2 | 0.5 | 0.4 | 2.7 | 1.7 |
| $C_2$ | | | Traces | | |
| $C_3$ | Trace | 3.2 | 4.1 | 5.1 | 3.8 |
| $C_4$ | 0.2 | 11.9 | 14.1 | 7.7 | 6.7 |
| Olefins $C_2$ | 2.0 | 8.5 | 5.2 | 10.3 | 11.1 |
| $C_3$ | 2.1 | 8.4 | 10.7 | 21.6 | 19.2 |
| $C_4$ | 1.4 | 10.8 | 12.1 | 15.5 | 15.1 |
| $C_5$+ aliphatics and aromatics | 1.5 | 56.6 | 53.3 | 37.0 | 42.3 |
| $C_2$ to $C_4$ olefins | 5.5 | 27.7 | 28.0 | 47.4 | 45.4 |

Example 34

This example illustrates the effect of space velocity on the methanol conversion to hydrocarbons. Table 5 hereafter indicates the results of this effect. The experimental and analytical procedures were similar to those of Example 32.

TABLE 5
Conversion of methanol to hydrocarbons: effect of space velocity

| | | | | | | |
|---|---|---|---|---|---|---|
| Feed | 28.5% methanol | | | | | |
| Pressure | Atmospheric | | | | | |
| Temperature | 450°C | | | | | |
| WHSV ($hr.^{-1}$) | 2.1 | 4.4 | 5.9 | 8.8 | 11.7 | 17.6 |
| % conv. methanol | 100 | 100 | 100 | 100 | 94.7 | 89.9 |
| Product distribution (wt.%) | | | | | | |
| Dimethyl ether | A | A | A | A | 0.4 | 0.9 |
| Paraffins | | | | | | |
| $C_1$ | 2.4 | 1.9 | 1.3 | 3.1 | 2.2 | 2.8 |
| $C_2$ | | | Traces | | | |
| $C_3$ | 3.5 | 0.9 | 0.7 | 0.6 | 0.7 | 0.4 |
| $C_4$ | 5.7 | 1.8 | 2.1 | 2.2 | 2.3 | 1.4 |
| Olefins | | | | | | |
| $C_2$ | 11.6 | 9.4 | 7.8 | 8.9 | 8.0 | 9.1 |
| $C_3$ | 25.9 | 43.7 | 42.9 | 39.8 | 41.1 | 41.3 |
| $C_4$ | 18.8 | 20.5 | 21.21 | 16.1 | 17.4 | 15.6 |
| $C_5$+aliphatic+aromatics | 32.2 | 21.8 | 23.9 | 29.5 | 28.3 | 29.5 |
| $C_2$ to $C_4$ olefins | 56.2 | 73.6 | 71.8 | 64.7 | 66.5 | 65.9 |

Example 35

The concentration of methanol in the feed greatly influences the formation of olefins in the conversion of methanol to hydrocarbons. This is illustrated in this examples and the results are tabulated in Table 6 herein. The methanol conversion and the analysis of the products were carried out as in Example 32.

TABLE 6

Conversion of methanol to hydrocarbons: effect of concentration of methanol in the feed

| | | | | |
|---|---|---|---|---|
| HS | | | | |
| WHSV (hr.$^{-1}$) | 4.4 | | | |
| Pressure | Atmospheric | | | |
| Temp. | 450°C | | | |
| Methanol concentration in the feed (% methanol in water v/v) | 100 | 71 | 44.3 | 28.5 |
| % conv. of methanol | 100 | 100 | 100 | 100 |
| Product distribution (wt.%) | | | | |
| Dimethyl ether | A | A | A | A |
| Paraffins | | | | |
| $C_1$ | 2.7 | 2.5 | 1.7 | 1.9 |
| $C_2$ | | Traces | | |
| $C_3$ | 5.1 | 3.5 | 1.4 | 0.9 |
| $C_4$ | 7.7 | 5.7 | 2.9 | 1.8 |
| Olefins | | | | |
| $C_2$ | 10.3 | 11.7 | 9.4 | 9.4 |
| $C_3$ | 21.6 | 25.9 | 37.6 | 43.7 |
| $C_4$ | 15.5 | 18.7 | 23.7 | 21.0 |
| $C_5$+aliphatics+aromatics | 37.0 | 32.2 | 23.3 | 21.8 |
| $C_2$ to $C_4$ olefins | 47.4 | 56.2 | 70.8 | 73.7 |

Example 36

The aging effect of encilite with time-on-stream of the feed during the conversion of methanol to hydrocarbons is given in this example. The experimental procedures and the analysis of the products were carried out as illustrated in Examples 32. The results are given in Table 7 hereafter.

TABLE 7
Conversion to methanol to hydrocarbons: effect on time on stream of the feed

| | | | | | |
|---|---|---|---|---|---|
| Catalyst weight | 3.2 gms | | | | |
| WHSV (hr.$^{-1}$) | 4.4 | | | | |
| Temperature | 450°C | | | | |
| Feed | 28.5% methanol 9 (v/v) | | | | |
| Cumulative feed in ml. | 350 | 980 | 1140 | 1350 | 1550 |
| Product distribution (Wt.%) | | | | | |
| Dimethyl ether | A | A | A | A | A |
| Paraffins | | | | | |
| $C_1$ | 1.9 | 1.9 | 1.4 | 1.5 | 1.7 |
| $C_2$ | | | Traces | | |
| $C_3$ | 0.9 | 0.9 | 0.8 | 0.9 | 0.9 |
| $C_4$ | 1.8 | 1.6 | 2.0 | 1.7 | 3.6 |
| Olefins | | | | | |
| $C_2$ | 9.4 | 9.7 | 8.4 | 8.0 | 9.7 |
| $C_3$ | 43.7 | 45.6 | 44.5 | 41.6 | 45.3 |
| $C_4$ | 20.6 | 20.0 | 21.0 | 17.2 | 17.0 |
| $C_5$+aliphatics+aromatics | 21.8 | 20.4 | 21.9 | 30.0 | 21.2 |
| $C_2$+$C_4$ olefins | 73.6 | 73.2 | 73.8 | 66.8 | 72.0 |

Example 37

This example illustrates the product distribution obtained when using aqueous methanol at a reaction temperature of 500°C. The methanol conversion and the analysis of the products are carried out as in Example 32.

TABLE 8
Conversion of methanol

| | | |
|---|---|---|
| Methanol concentration in the feed (% methanol in water V/V) | 71 | |
| Pressure | Atmospheric | |
| Temperature °C | 500 | |
| WHSV | 2.2 | 4.4 |
| % conversion of methanol | 100 | 100 |
| Product distribution (% Wt.) | | |
| Dimethyl ether | 0 | 0 |
| Paraffins | | |
| $C_1$ | 5.5 | 5.8 |
| $C_2$ | A | A |
| $C_3$ | 2.9 | 1.8 |
| $C_4$ | 3.1 | 1.8 |
| Olefins | | |
| $C_2$ | 14.7 | 16.0 |
| $C_3$ | 27.4 | 30.5 |
| $C_4$ | 18.4 | 15.8 |
| $C_5$+hydrocarbons | 28.1 | 28.3 |
| $C_2$ to $C_4$ olefins | 60.5 | 62.3 |

Example 38

This example illustrates the product distribution obtained when using ethyl, propyl and butyl alcohols as the feedstock. The alkyl alcohol conversion and the analysis of the products are carried out as in Example 32.

TABLE 9
Conversion of alkyl alcohols

| | | | |
|---|---|---|---|
| Temperature | 450°C | | |
| Pressure | Atmospheric | | |
| WHSV ($hr.^{-1}$) | 2.2 | | |
| Feed | Pure (100%) alcohol | | |
| Alcohol | *ethanol* | *propanol* | *butanol* |
| Conversion of alcohol (%) | 100 | 100 | 100 |
| Product distribution (Wt.%) | | | |
| Paraffins | | | |
| $C_1$ | 2.5 | 3.1 | 2.1 |
| $C_2$ | 0.7 | 0.4 | 0.8 |
| $C_3$ | 4.5 | 3.8 | 5.1 |
| $C_4$ | 8.9 | 7.7 | 9.0 |
| Olefins | | | |
| $C_2$ | 18.5 | 14.1 | 12.5 |
| $C_3$ | 20.2 | 23.8 | 21.8 |
| $C_4$ | 17.0 | 19.5 | 21.2 |
| $C_5$+aliphatics | 20.4 | 18.0 | 10.5 |
| Aromatics | 7.3 | 9.7 | 16.9 |

**Claims**

1. A crystalline encilite characterised in that it shows an X-ray powder diffraction pattern having reflection lines as shown below:

| d | I/Ig |
|---|---|
| 11.0420 | 83.30 |
| 9.9945 | 62.57 |
| 9.7096 | 15.90 |
| 7.4057 | 10.22 |
| 7.0752 | 6.93 |
| 6.6713 | 7.95 |
| 6.3203 | 11.36 |
| 5.9804 | 10.22 |
| 5.6755 | 9.19 |
| 5.5345 | 9.09 |
| 5.3679 | 3.40 |
| 5.1038 | 3.40 |
| 4.9087 | 5.68 |
| 4.5950 | 6.25 |
| 4.4357 | 3.40 |
| 4.3496 | 9.09 |
| 4.2467 | 15.99 |
| 4.0733 | 5.68 |
| 4.0080 | 9.09 |
| 3.8371 | 100.00 |
| 3.7416 | 34.09 |
| 3.7047 | 46.59 |
| 3.6449 | 31.81 |
| 3.4293 | 11.36 |
| 3.3285 | 5.50 |
| 3.2406 | 4.54 |
| 3.1291 | 2.84 |
| 3.0394 | 10.79 |
| 2.9378 | 5.60 |

where d is the interplanar spacing (Angstroms) and I/Ig are the relative intensity values; in that it gives infrared framework vibration frequencies as shown below:

| Frequency ($cm^{-1}$) | Intensity |
|---|---|
| 455 | MS |
| 550 | MS |
| 590 | M |
| 620 | M |
| 678 | M |
| 730 | M |
| 800 | MS |
| 870 | MS |
| 888 | MS |
| 1040 | S |
| 1100 | S |
| 1230 | MS |

where

| | | |
|---|---|---|
| VW | (very weak) | Less than 10% absorption |
| W | (weak) | 10—19% absorption |
| M | (medium) | 20—39% absorption |
| MS | (medium strong) | 40—69% absorption |
| S | (strong) | 70—89% absorption; |

and in that the formula representing the composition of the encilite, expressed in moles of the oxides, is

$$1.0\pm0.2\ M_2O:\ Fe_2O_3:\ 30\text{—}300\ SiO_2:\ ZH_2O,$$

where M is a monovalent cation and Z is a value from 0 to 20.

2. A crystalline encilite as claimed in claim 1, characterised in that M is sodium, ammonium, hydrogen or a mixture thereof.

3. A process for the preparation of a crystalline encilite as claimed in claim 1 or claim 2, comprising reacting in aqueous medium an iron compound, a silicon compound, and a tetraalkyl ammonium salt,

characterised in that the aqueous medium also contains sulphuric acid, and in that the resulting gel is maintained at a temperature between 100—200°C for a period of 1 to 15 days.

4. A process as claimed in claim 3, characterised in that the aqueous mixture comprises sodium compound and in that the tetraalkyl ammonium salt comprises triethyl-n-propyl ammonium bromide, tetrapropyl ammonium bromide or triethyl-n-butylammonium bromide.

5. A process as claimed in claim 3 or claim 4, characterised in that the silicon compound content of said aqueous medium comprises silicic acid, colloidal silica, fumed silica, chromatographic silica, silica gel or sodium silicate.

6. A process as claimed in any one of claims 3 to 5, characterised in that the iron compound source comprises ferric sulphate hexahydrate or ferric nitrate.

7. A process as claimed in any one of claims 3 to 6, characterised by the further step of subjecting the product thereof to calcination.

8. A process as claimed in claim 7, characterised in that the calcined encilite is subjected to ion exchange with an ammonium salt to obtain encilite having a molar ratio of sodium oxide to iron oxide in the range 0.05 to 0.3.

9. A process as claimed in any one of claims 3 to 8, characterised in that the reaction mixture has a composition in terms of mole ratios of oxides falling within the following range:

| | |
|---|---|
| $SiO_2/Fe_2O_3$ | — 30—300 |
| $Na_2O/Fe_2O_3$ | — 2—10 |
| Org. compd./$Fe_2O_3$ | — 1—20 |
| $H_2SO_4/Fe_2O_3$ | — 1—5 |
| $H_2O/Fe_2O_3$ | —500—5000 |

10. A process as claimed in claim 8 or claim 9, characterised in that it is further calcined in air to yield a hydrogen ion solid composite.

11. A method for converting an alkanol or an aqueous alkanol mixture into hydrocarbons consisting primarily of light olefins, characterised in that it comprises contacting in the vapour phase under a conversion reaction temperature from 300°C to 500°C, a feed comprising one or more monohydric alcohols having 1 to 4 carbon atoms, or a mixture thereof with water over a crystalline encilite produced by a process as claimed in claim 10.

12. A method as claimed in claim 11, characterised in that said encilite catalyst is formulated with a binder in an amount from 50% to 95% by weight in order to impart greater mechanical strength.

13. A method as claimed in claim 11 or claim 12, characterised in that said encilite catalyst is blended with alumina, bentonite or kaolinite and water to the desired consistence and formed into any convenient shape, said shape being dried, being employed as the catalyst in the conversion process.

14. A method as claimed in any one of claims 11 to 13, characterised in that the encilite catalyst is located within a downflow integral fixed bed reactor and alkanol vapour is passed over it at a weight hourly space velocity (WHSV) of 2 to 20.

15. A method as claimed in any one of claims 11 to 14, characterised in that the mixture of alkanol and water contains from 28% to 71% by volume alkanol.

16. A method as claimed in any one of claims 11 to 15, characterized in that the feed alkanol is methanol.

**Patentansprüche**

1. Kristalliner Enzilith, dadurch gekennzeichnet, daß er ein Röntgenpulverbeugungsdiagramm mit im folgenden dargestellten Reflektionslinien zeigt:

| d | I/Ig |
|---|---|
| 11,0420 | 83,30 |
| 9,9945 | 62,57 |
| 9,7096 | 15,90 |
| 9,4057 | 10,22 |
| 7,0752 | 6,93 |
| 6,6713 | 7,95 |
| 6,3203 | 11,36 |
| 5,9804 | 10,22 |
| 5,6755 | 9,19 |
| 5,5345 | 9,09 |
| 5,3679 | 3,40 |
| 5,1038 | 3,40 |
| 4,9087 | 5,68 |
| 4,5950 | 6,25 |
| 4,4357 | 3,40 |
| 4,3496 | 9,09 |
| 4,2467 | 15,99 |
| 4,0733 | 5,68 |
| 4,0080 | 9,09 |
| 3,8371 | 100,00 |
| 3,7416 | 34,09 |
| 3,7047 | 46,59 |
| 3,6449 | 31,81 |
| 3,4293 | 11,36 |
| 3,3285 | 5,50 |
| 3,2406 | 4,54 |
| 3,1291 | 2,84 |
| 3,0394 | 10,79 |
| 2,9378 | 5,60 |

wobei d der Abstand zwischen den Ebenen (in Ångström) ist und I/Ig die relativen Intensitätswerte sind, daß sich wie im folgenden gezeigte, infrarote Gerüstschwingungsfrequenzen ergeben:

| Frequenz ($cm^{-1}$) | Intensität |
|---|---|
| 455 | MS |
| 550 | MS |
| 590 | M |
| 620 | M |
| 678 | M |
| 730 | M |
| 800 | MS |
| 870 | MS |
| 888 | MS |
| 1040 | S |
| 1100 | S |
| 1230 | MS |

wobei

| | | |
|---|---|---|
| VW | (sehr schwach) | Weniger als 10% Abs. |
| W | (schwach) | 10—19% Absorption |
| M | (mittel) | 20—39% Absorption |
| MS | (mittelstark) | 40—69% Absorption |
| S | (stark) | 70—89% Absorption, |

und daß die der Zusammensetzung des Einzilithen entsprechende Formel, in Molen der Oxide ausgedrückt,

$$1{,}0 \pm 0{,}2\ M_2O:\ Fe_2O_3:\ 30\text{—}300\ SiO_2:\ ZH_2O \text{ ist,}$$

wobei M ein einwertiges Kation ist und Z ein Wert von 0 bis 20.

2. Kristalliner Enzilith nach Anspruch 1, dadurch gekennzeichnet, daß M Natrium, Ammonium, Wasserstoff oder ein Gemisch davon bedeutet.

3. Verfahren zur Herstellung eines kristallinen Enzilithen nach Anspruch 1 oder Anspruch 2, wobei man

in wäßrigem Medium eine Eisenverbindung, eine Siliziumverbindung und ein Tetraalkylammoniumsalz reagieren läßt, dadurch gekennzeichnet, daß das wäßrige Medium auch Schwefelsäure enthält und daß das entstehende Gel bei einer Temperatur zwischen 100—200°C über eine Dauer von 1 bis 15 Tagen aufrechterhalten bleibt.

4. Verfahren nach Anspruch 3, dadurch gekennzeichnet, daß das wäßrige Gemisch eine Natriumverbindung enthält und daß das Tetraalkylammoniumsalz Triethyl-n-propylammoniumbromid, Tetrapropylammoniumbromid oder Triethyl - n - propylammoniumbromid ist.

5. Verfahren nach Anspruch 3 oder Anspruch 4, dadurch gekennzeichnet, daß der Gehalt an Siliziumverbindung von besagtem wäßrigem Medium aus Kieselsäure, kolloidaler Kieselsäure, Kieselsäurerauch chromatografischer Silika, Kieselgel oder Natriumsilikat besteht.

6. Verfahren nach einem der Ansprüche 3 bis 5, dadurch gekennzeichnet, daß die Eisenverbindungsquelle Eisen - (III) - sulfathexahydrat oder Ferrinitrat ist.

7. Verfahren nach einem der Ansprüche 3 bis 6, dadurch gekennzeichnet, daß als weiterer Schritt das Entstehungsprodukt davon einer Röstphase ausgesetzt wird.

8. Verfahren nach Anspruch 7, dadurch gekennzeichnet, daß der geröstete Enzilith einem Ionenaustausch mit einem Ammoniumsalz unter Erhalt von Enzilith mit einem Molverhältnis von Natriumoxid zu Eisenoxid im Bereich von 0,05 bis 0,3 ausgesetzt wird.

9. Verfahren nach einem der Ansprüche 3 bis 8, dadurch gekennzeichnet, daß das Reaktionsgemisch eine Zusammensetzung bezüglich der Molverhältnisse von Oxiden aufweist, die innerhalb den folgenden Bereich fallen:

| | |
|---|---|
| $SiO_2/Fe_2O_3$ | — 30—300 |
| $Na_2O/Fe_2O_3$ | — 2—10 |
| Org. Verb./$Fe_2O_3$ | — 1—20 |
| $H_2SO_4/Fe_2O_3$ | — 1—5 |
| $H_2O/Fe_2O_3$ | —500—5000. |

10. Verfahren nach Anspruch 8 oder Anspruch 9, dadurch gekennzeichnet, daß es weiter an Luft geröstet wird, um einen Wasserstoffion-Festverbundstoff zu erhalten.

11. Methode zur Umwandlung eines Alkanols oder eines wäßrigen Alkanolgemisches in primär aus leichten Olefinen bestehende Kohlenwasserstoffe, dadurch gekennzeichnet, daß man in der Dampfphase unter einer Umwandlungsreaktionstemperatur von 300°C bis 500°C eine Einspeisung aus einem oder mehreren einwertigen Alkoholen mit 1 bis 4 Kohlenstoffatomen, oder ein Gemisch davon mit Wasser, über einem kristallinen, durch eine Verfahren nach Anspruch 10 hergestellten Enzilithen in Berührung bringt.

12. Methode nach Anspruch 11, dadurch gekennzeichnet, daß der Ansatz für besagten Enzilithkatalysator ein Bindemittel in einer Menge von 50 bis 95 Gewichtsprozenten zur Verleihung einer höheren mechanischen Festigkeit enthält.

13. Methode nach Anspruch 11 oder Anspruch 12, dadurch gekennzeichnet, daß besagter Enzilithkatalysator mit Tonerde, Bentonit oder Kaolinit und Wasser zur gewünschten Beschaffenheit vermengt und in eine beliebige geeignete Form gebracht wird, wobei jene Form getrocknet wird und als Katalysator im Umwandlungsprozess verwendet wird.

14. Methode nach einem der Ansprüche 11 bis 13, dadurch gekennzeichnet, daß der Enzilithkatalysator innerhalb eines Rücklaufstrom-Festbettreaktors angeordnet ist und Alkanoldampf darüber bei einer stündlichen Gewichtsraumgeschwindigkeit (WHSV) von 2 bis 20 geleitet wird.

15. Methode nach einem der Ansprüche 11 bis 14, dadurch gekennzeichnet, daß das Gemisch aus Alkanol und Wasser 28 bis 71 Volumenprozent Alkanol enthält.

16. Methode nach einem der Ansprüche 11 bis 15, dadurch gekennzeichnet, daß die Alkanoleinspeisung Methanol ist.

**Revendications**

1. Une encilite cristalline caractérisée en ce qu'elle montre un motif de diffraction de la poudre aux rayons X présentant des lignes de réflexion telles qu'elles sont représentées ci-dessous:

| d | I/Ig |
|---|---|
| 11,0420 | 83,30 |
| 9,9945 | 6257 |
| 9,7096 | 15,90 |
| 7,4057 | 10,22 |
| 7,0752 | 6,93 |
| 6,6713 | 7,95 |
| 6,3203 | 11,36 |
| 5,9804 | 10,22 |
| 5,6755 | 9,19 |
| 5,5345 | 9,09 |
| 5,3679 | 3,40 |
| 5,1038 | 3,40 |
| 4,9087 | 5,68 |
| 4,5950 | 6,25 |
| 4,4357 | 3,40 |
| 4,3496 | 9,09 |
| 4,2467 | 15,99 |
| 4,0733 | 5,68 |
| 4,0080 | 9,09 |
| 3,8371 | 100,00 |
| 3,7416 | 34,09 |
| 3,7047 | 46,59 |
| 3,6449 | 31,81 |
| 3,4293 | 11,36 |
| 3,3285 | 5,50 |
| 3,2406 | 4,54 |
| 3,1291 | 2,84 |
| 3,0394 | 10,79 |
| 2,9378 | 5,60 |

où d'est la distance interplanaire (en angstroms) et I/Ig sont les valeurs d'intensité relatives; en ce qu'elle donne des fréquences de vibration de base en infrarouge telles qu'elles sont représentées ci-dessous.

| Frequence ($cm^{-1}$) | Intensite |
|---|---|
| 455 | MS |
| 550 | MS |
| 590 | M |
| 620 | M |
| 678 | M |
| 730 | M |
| 800 | MS |
| 870 | MS |
| 888 | MS |
| 1040 | S |
| 1100 | S |
| 1230 | MS |

ou

| | | |
|---|---|---|
| TF | (très faible) | Moins de 10% d'absorption |
| F | (faible) | 10—19% d'absorption |
| M | (moyenne) | 20—39% d'absorption |
| MI | (moyenne intense) | 40—69% d'absorption |
| I | (intense) | 70—89% d'absorption; |

et en ce que la formule représentant la composition de l'encilite, exprimée en moles des oxydes, est

$$1{,}0\pm0{,}2\ M_2O:\ Fe_2O_3:\ 30—300\ SiO_2:\ ZH_2O,$$

où M est un cation monovalent et Z est une valeur comprise entre 0 et 20.

2. Une encilite cristalline selon la revendication 1, caractérisée en ce que M est du sodium, de l'ammonium, de l'hdyrogène ou un mélange de ceux-ci.

3. Un procédé pour la préparation d'une encilite cristalline selon la revendication 1 ou la revendication

2, comprenant la réaction en milieu aqueux d'un composé de fer, un composé de silicium, et un sel de tétraalkyleammonium, caractérisé en ce que le milieu aqueux contient également de l'acide sulfurique, et en ce que le gel résultant est maintenu à une température comprise entre 100 et 200°C pendant une période de 1 à 15 jours.

4. Un procédé selon la revendication 3, caractérisé en ce que le mélange aqueux comprend un composé du sodium et en ce que le sel de tétraalkyleammonium comprend du bromure de triéthyl - n - propylammonium, du bromure de tétrapropylammonium ou du bromure de triéthyl - n - butylammonium.

5. Un procédé selon la revendication 3 ou la revendication 4, caractérisé en ce que le contenu en composé de silicium dudit milieu aqueux comprend de l'acide silicique, de la silice colloidale, de la silice de combustion, de la silice chromatographique, du gel de silice ou du silicate de sodium.

6. Un procédé selon l'une quelconque des revendications 3 à 5, caractérisé en ce que la source de composé de fer comprend du sulfate ferrique hexahydraté ou du nitrate ferrique.

7. Un procédé selon l'une quelconque des revendications 3 à 6, caractérisé par l'étape supplémentaire de soumettre le produit de celui-ci à une calcination.

8. Un procédé selon la revendication 7, caractérisé en ce que l'encilite calcinée est soumise à une échange d'ions avec un sel d'ammonium pour obtenir de l'encilite ayant un rapport molaire de l'oxyde de sodium à l'oxyde de fer compris dans l'intervalle de 0.05 à 0.3.

9. Un procédé selon l'une quelconque des revendications 3 à 8, caractérisé en ce que le mélange réactionnel a une composition en termes de rapports molaires d'oxydes tombant dans l'intervalle suivant:

| | |
|---|---|
| $SiO_2/Fe_2O_3$ | — 30—300 |
| $Na_2O/Fe_2O_3$ | — 2—10 |
| Composé organique/$Fe_2O_3$ | — 1—20 |
| $H_2SO_4/Fe_2O_3$ | — 1—5 |
| $H_2OO/Fe_2O_3$ | —500—5000 |

10. Un procédé selon la revendication 8 ou la revendication 9, caractérisé en ce qu'il est en outre calciné à l'air pour donner un composite solide d'ions d'hydrogène.

11. Une méthode pour transformer un alcanol ou un mélange d'alcanol aqueux en des hydrocarbures constitutés principalement d'oléfines légères, caractérisée en ce qu'elle comprend la mise en contact en phase vapeur à une température de réaction de transformation comprise entre 300°C et 500°C, une charge d'alimentation comprenant un ou plusieurs alcools monohydriques ayant de 1 à 4 atomes de carbone ou un mélange de ceux-ci avec de l'eau sur une encilite cristalline produite par un procédé selon la revendication 10.

12. Une méthode selon la revendication 11, caractérisée en ce que ledit catalyseur d'encilite est formulé avec un liant en quantité comprise entre 50% et 95% en poids afin de lui conférer une plus grande résistance mécanique.

13. Une méthode selon la revendication 11 ou la revendication 12, caractérisée en ce que ledit catalyseur d'encilite est mélangé à de l'alumine, de la bentonite ou de la kaolinite et de l'eau, jusqu'à la consistance désirée, et mis sous toute forme convenable quelconque, ladite forme étant séchée, et étant employée en tant que catalyseur dans le procédé de transformation.

14. Une méthode selon l'une quelconque des revendications 11 à 13, caractérisée en ce que le catalyseur d'encilite est placé dans une réacteur à lit fix intégral à écoulement vers le bas et en ce que l'on fait passer de la vapeur d'alcanol sur lui à une vitesse spatiale horaire pondérale (VSHP) comprise entre 2 et 20.

15. Une méthode selon l'une quelconque des revendications 11 à 14, caractérisée en ce que le mélange d'alcanol et d'eau contient 28 à 71% en volume d'alcanol.

16. Une méthode selon l'une quelconque des revendications 11 à 15, caractérisée en ce que l'alcanol d'alimentation est du méthanol.